Europäisches Patentamt
European Patent Office
Office européen des brevets

(11) Veröffentlichungsnummer: **0 210 530 A1**

# (12) EUROPÄISCHE PATENTANMELDUNG

(21) Anmeldenummer: 86109671.7

(22) Anmeldetag: 15.07.86

(51) Int. Cl.[4]: C 07 D 237/04
A 61 K 31/50

(30) Priorität: 27.07.85 DE 3527036
23.05.86 DE 3617383

(43) Veröffentlichungstag der Anmeldung:
04.02.87 Patentblatt 87/6

(84) Benannte Vertragsstaaten:
AT BE CH DE FR GB IT LI NL SE

(71) Anmelder: Merck Patent Gesellschaft mit beschränkter Haftung
Frankfurter Strasse 250
D-6100 Darmstadt(DE)

(72) Erfinder: Jonas, Rochus, Dr.
Kesselhutweg 12
D-6100 Darmstadt(DE)

(72) Erfinder: Piulats, Jaime, Dr.
Rvdo. Quintin Mallofre 2
ES-08030-Barcelona(ES)

(72) Erfinder: Lues, Ingeborg, Dr.
Paul Wagnerstrasse 13
D-6100 Darmstadt(DE)

(72) Erfinder: Schliep, Hans-Jochen, Dr.
Weingartenstrasse 16
D-6109 Traisa(DE)

## (54) 6-Arylalkenylpyridazinone.

(57) Neue 6-Arylalkenylpyridazinone der allgemeinen Formel I,

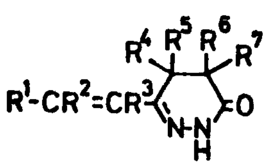

I

worin

R bis $R^6$ die in Patentanspruch 1 angegebene Bedeutung haben, sowie deren Salze zeigen positiv inotrope Wirkungen.

EP 0 210 530 A1

Croydon Printing Company Ltd

Merck Patent Gesellschaft
mit beschränkter Haftung

6100 D a r m s t a d t

# 6-Arylalkenylpyridazinone

Die Erfindung betrifft neue 6-Arylalkenylpyridazinone der allgemeinen Formel I,

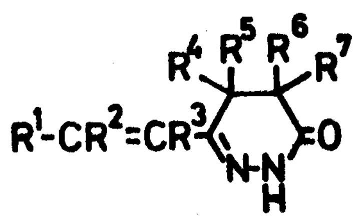

I

worin

$R^1$ eine Phenylgruppe oder eine ein- bis vierfach durch R, OH, SH, OR, SR, SO-R, $SO_2$-R, O-$SO_2$-R, S-$SO_2$-R, $NH_2$, NH-R, $NR_2$, NH-CO-R, F, Cl, Br, J, $CF_3$, $NO_2$, CN und/oder CO-$NH_2$ substituierte Phenylgruppe,

R Alkyl mit 1 bis 6 C-Atomen, Alkenyl oder Alkinyl mit 2 bis 6 C-Atomen,

$R^2$ und $R^3$ H oder Alkyl mit 1 bis 3 C-Atomen,

$R^4$ und $R^7$ jeweils H oder Alkyl mit 1 bis 4 C-Atomen und

$R^5$ und $R^6$ jeweils H oder zusammen eine C-C-Bindung bedeuten,

worin ferner

$R^1$ nur dann p-Tolyl oder p-Methoxyphenyl sein kann, wenn einer der Reste $R^2$ bis $R^7$ von H verschieden ist,

sowie ihre Salze.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I bei guter Verträglichkeit wertvolle pharmakologische Eigenschaften besitzen. So zeigen sich beispielsweise Wirkungen auf den Kreislauf, insbesondere positiv inotrope, blutdrucksenkende und herzentlastende Wirkungen.

Die positiv inotrope Wirkung kann in vitro am barbiturat-insuffizienten Herz-Lungen-Präparat des Meerschweinchens (Methode vgl. H.J. Schliep, et al., 8th Internat. Congress of Pharmacology, Tokyo 1981, Abstr. of papers No. 923) sowie im in-vivo-Versuch als Zunahme der linksventrikulären Druckanstiegsgeschwindigkeit des barbituratnarkotisierten Hundes bei intravenöser Gabe (W. Diederen und R. Kadatz, Arzneimittelforschung 31 (I), 146 - 150, 1981) und des wachen, mit einem Konigsberg-Aufnehmer instrumentierten Hundes (Methode vgl. J. Mahler et al., Circulation 50, 720 - 727, 1974) bei oraler Gabe beobachtet werden.

Die Substanzen vermindern den an der Carotisschlinge des wachen mischrassigen Hundes gemessenen Blutdruck (Methodik vgl. E.C. van Leersum, Pflügers Archiv 142, 377 - 395, 1911) bei nephrogen hypertonen Tieren (Methodik vgl. I.H. Page, Science 89, 273 - 274, 1939) im 10-Tage-Dauerversuch bei oraler Gabe von Dosen, die niedriger als 2 mg/kg/Tier liegen können, dosisabhängig auf ein erniedrigtes Niveau.

Weiterhin wird der bei katheter-tragenden (Methode vgl. J.R. Weeks und J.A. Jones, Proc.Soc.Exptl.Biol.Med. 104, 646 - 648, 1960) wachen spontan hypertonen Ratten (Stamm SHR/NIH/-MO/CHB-EMD) direkt gemessene arterielle Blutdruck nach einmaliger intragastraler Gabe ab 10 mg/kg dosisabhängig gesenkt. Auch an der narkotisierten Katze können die blutdrucksenkenden Wirkungen nach intravenöser Applikation durch direkte Messung des Carotisdruckes verifiziert werden.

Die herzentlastende Wirkung läßt sich aus der am narkotisierten Hund nach intravenöser Gabe beobachteten Zunahme der gesamt-vaskulären Kapazität (H. Suga et al., Pflügers Archiv 361, 95 - 98, 1975) ableiten.

Weiterhin treten antithrombotische, thrombozytenaggregationshemmende und die Erythrozytenform beeinflussende Eigenschaften auf. Die Beeinflussung der Thrombozytenfunktion im Sinne einer Aggregationshemmung kann an der Ratte ex vivo im Test nach Born (Nature 194, 927 - 929, 1962) nachgewiesen werden. Die antithrombotische Wirkung zeigt sich in der Verlängerung der Blutungszeit nach

Stella (Thrombos. Res. 7, 709 - 716, 1975) in der Verminderung des Thrombusgewichtes bei der kälteinduzierten Thrombosierung der Jugular-Vene bei der Ratte nach Meng (Ther. Ber. 47, 69 - 79, 1975) und der Erhöhung der zur vollständigen Thrombosierung notwendigen Laserimpulse an der Mesenterial-Venole der Ratte, entsprechend einer Abwandlung der Methode nach Kovacs (Microvasc. Res. 6, 194 - 201, 1973). Die günstige Wirkung auf die Erythrozytenverformbarkeit ist im Nucleoporefilter nach Schmid-Schönbein (Pflüger's Archiv 338, 93 - 114, 1973) nachweisbar. Auch lassen sich günstige Effekte auf die Fibrinolyse/Euglobulinlysiszeit nach v. Kaulla (Progr. Chem. Fibrinol. Thrombol. 1, 131 - 149, 1975; ed. J.F. Davidson, Raven Press, N.Y.) feststellen.

Die Verbindungen der Formel I können daher als Arzneimittel in der Human- und Veterinärmedizin verwendet werden. Ferner können sie als Zwischenprodukte zur Herstellung weiterer Arzneimittelwirkstoffe verwendet werden.

Gegenstand der Erfindung sind die 6-Arylalkenylpyridazinone der Formel I.

In dieser Formel bedeutet R vorzugsweise Alkyl mit 1 bis 4 C-Atomen, insbesondere Methyl. Der Rest $R^1$ bedeutet vorzugsweise eine durch R, OH, OR, SR, SO-R, $SO_2$-R, F, Cl,Br, J und/oder $CF_3$, insbesondere durch OR, SR, SO-R, $SO_2$-R und $CF_3$ ein- oder zweifach substituierte Phenylgruppe. Besonders bevorzugt ist $R^1$ eine Phenylgruppe, die einen schwefelhaltigen Substituenten (insbesondere SR, SO-R oder $SO_2$-R) trägt und (vorzugsweise einen) weitere(n) Substituenten (insbesondere Cl, ferner F, Br oder J, weiterhin R oder OR) tragen kann. Bevorzugte Bedeutungen von $R^2$ und $R^3$ sind jeweils H oder Methyl, insbesondere $R^2 = R^3 = H$. Die Reste $R^4$ und $R^7$ bedeuten

vorzugsweise jeweils H oder Methyl, insbesondere $R^4 = CH_3$ und $R^7 = H$. Die für die Reste $R^5$ und $R^6$ bevorzugte Bedeutung ist jeweils H.

Bei mehrfacher Substitution des Benzolrings sind die Substituenten vorzugsweise verschieden, sie können aber auch gleich sein. Sie befinden sich vorzugsweise in 4- und/oder 2-Stellung, sie können aber auch in 3-, 5- oder 6-Stellung stehen. Die durch die Reste $R^2$ und $R^3$ substituierte Doppelbindung besitzt vorzugsweise die trans-Konfiguration, sie kann jedoch auch cis-konfiguriert sein.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Id ausgedrückt werden, die der Formel I entsprechen, worin jedoch

in I a $R^1$ eine Phenylgruppe bedeutet, die einfach durch SH, SR, SO-R, $SO_2$-R oder $SSO_2$-R substituiert ist und zusätzlich ein- bis dreifach durch R, OH, SH, OR, SR, SO-R, $SO_2$-R, O-$SO_2$-R, S-$SO_2$-R, $NH_2$, NH-CO-R, F, Cl, Br, J, $CF_3$, $NO_2$, CN und/oder $CONH_2$ substiuiert sein kann;

in I b $R^1$ eine durch eine SR-, SO-R- oder $SO_2$-R-Gruppe und zusätzlich durch F, Cl, Br oder J substituierte Phenylgruppe bedeutet;

in I c $R^1$ 2-Chlor-4-methylthiophenyl, 2-Chlor-4-methylsulfinylphenyl oder 2-Chlor-4-methylsulfonylphenyl bedeutet;

in I d $R^1$ 2-Chlor-4-methylsulfinylphenyl bedeutet.

Besonders bevorzugt sind Verbindungen der Formeln I' sowie Ia' bis Id', die den Formeln I sowie Ia bis Id entsprechen und worin zusätzlich $R^2$ und $R^3$ jeweils H bedeuten.

Unter diesen sind besonders bevorzugt Verbindungen der Formeln I" sowie Ia" bis Id", die den Formeln I' sowie Ia' bis Id' entsprechen und worin zusätzlich $R^4$ H oder Methyl (insbesondere Methyl) und $R^7$ H bedeuten.

Von allen Verbindungen der Formeln I, Ia bis Id, I', Ia' bis Id', I" sowie Ia" bis Id" sind diejenigen bevorzugt, in denen $R^5$ und $R^6$ jeweils H bedeuten.

Gegenstand der Erfindung ist ferner ein Verfahren zur Herstellung der 6-Arylalkenylpyridazinone der Formel I, dadurch gekennzeichnet, daß man eine Carbonsäure der allgemeinen Formel II,

$$R^1\text{-}CR^2\text{=}CR^3\text{-}CO\text{-}CR^4R^5\text{-}CR^6R^7\text{-}COOH \qquad II$$

worin $R^1$ bis $R^7$ die bei Formel I angegebene Bedeutung haben,

oder ein reaktionsfähiges Derivat einer solchen Carbonsäure mit Hydrazin oder einem reaktionsfähigen Hydrazinderivat umgesetzt,

oder daß man ein 1,2-Diketon der allgemeinen Formel III,

$$R^1\text{-}CR^2\text{=}CR^3\text{-}CO\text{-}CO\text{-}R^4 \qquad III$$

worin $R^1$ bis $R^4$ die bei Formel I angegebene Bedeutung haben,
mit Hydrazin und Essigsäure oder Acethydrazid umsetzt,

oder daß man ein Hydrazon der allgemeinen Formel IV,

$$R^1-CR^2=CR^3-C(=N-NH_2)-CO-R^4 \qquad IV$$

worin $R^1$ bis $R^4$ die bei Formel I angegebene Bedeutung haben,
mit Essigsäure umsetzt,

oder daß man eine Oxoverbindung der allgemeinen Formel V mit einem Phosphonat der allgemeinen Formel VI,

$R^1-CO-R^2$ V

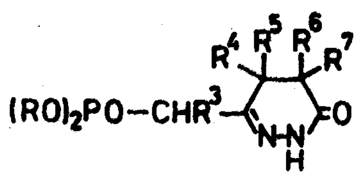

VI

worin R bis $R^7$ die bei Formel I angegebene Bedeutung haben, umsetzt,

und/oder daß man Thioether der Formel I, worin mindestens einer der Substituenten SR bedeutet, zu den entsprechenden Sulfoxiden oder Sulfonen oxidiert,

und daß man gegebenenfalls in ein erhaltenes Dihydropyridazinon der Formel I ($R^5$ = $R^7$ = H) durch Behandeln mit dehydrierenden Mitteln eine Doppelbindung in 4(5)-Stellung einführt und/oder daß man eine basische Verbindung der Formel I mit einer Säure in das zugehörige Säureadditionssalz überführt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formel II können nach an sich bekannten Methoden hergestellt werden, beispielsweise durch Umsetzung von Benzaldehyden oder Alkylphenonen mit Lävulinsäure oder ihren Derivaten.

Anstelle der Carbonsäuren der Formel II können auch ihre reaktionsfähigen Derivate in die erfindungsgemäße Reaktion eingesetzt werden. Als solche eignen sich insbesondere die Ester, z.B. die Alkylester, worin die Alkylgruppe vorzugsweise 1 - 4 C-Atome besitzt, insbesondere die Methyl- und Ethylester. Des weiteren eignen sich z.B. die Nitrile der Säuren der Formel II. Weiterhin können z.B. die Säurehalogenide der Säuren der Formel II verwendet werden, z.B. die Säurechloride oder Säurebromide. Weitere geeignete reaktionsfähige Derivate der Carbonsäuren der Formel II können während der Reaktion in situ gebildet werden, ohne daß man sie isoliert. Dazu gehören beispielsweise die Hydrazone der Formel $R^1-CR^2=CR^3-C(=N-NH_2)-CR^4R^5-CR^6R^7-COOH$ und die Hydrazide der Formel $R^1-CR^2=CR^3-CO-CR^4R^5-CR^6R^7-CO-NH-NH_2$.

Die Ausgangsstoffe der Formeln III und IV können nach an sich bekannten Methoden hergestellt werden, beispielsweise durch Umsetzung von Benzaldehyden oder Alkylphenonen mit Mesoxalaldehydsäure oder ihren Derivaten oder Homologen

zu Verbindungen der Formel III oder mit Mesoxalaldehydsäurehydrazon oder dessen Derivaten oder Homologen zur Verbindungen der Formel IV.

Als reaktionsfähige Derivate des Hydrazins eignen sich z.B. Hydrazinhydrat, Acethydrazid, Semicarbazid oder Carbazinsäureester.

Für die Umsetzung mit den Carbonsäuren der Formel II verwendet man vorteilhaft ein Äquivalent an Hydrazin bzw. einem reaktionsfähigen Hydrazinderivat, das gleichzeitig als Lösungsmittel dienen kann. Zweckmäßiger ist es jedoch, ein zusätzliches inertes Lösungsmittel zuzusetzen. Als inerte Lösungsmittel eignen sich vorzugsweise Alkohole wie Methanol, Ethanol, Isopropanol, n-Butanol, Isoamylalkohol, Glykole und deren Ether wie Ethylenglykol, Diethylenglykol, Ethylenglykolmonomethyl- oder -monoethylether (Methylglykol oder Ethylglykol), ferner Ether, insbesondere wasserlösliche Ether wie Tetrahydrofuran, Dioxan oder Ethylenglykoldimethylether (Diglyme); ferner Wasser sowie Gemische dieser Lösungsmittel untereinander, insbesondere Gemische mit Wasser, z.B. wässriges Ethanol. Die Reaktionstemperaturen liegen zweckmäßig zwischen etwa 20 und 200 °C, vorzugsweise zwischen 20 und 100 °C, die Reaktionszeiten zwischen etwa 1 und 48 Stunden.

Für die Umsetzung mit 1,2-Diketonen der allgemeinen Formel III können die gleichen Lösungsmittel und Reaktionsbedingungen angewendet werden, vorausgesetzt dem Reaktionsgemisch wird eine der angewandten Menge Hydrazin mindestens äquivalente Menge Essigsäure zugesetzt, wobei sich die Essigsäure vorzugsweise im Überschuß befindet. Besonders vorteilhaft werden 1,2-Diketone der Formel III mit Acethydrazid zu den erfindungsgemäßen Arylalkenylpyridazinonen der Formel I umgesetzt. Die Umsetzung erfolgt vorteilhaft mit einem Überschuß an Acethydrazid, der gleichzeitig als Lösungsmittel dienen kann. Es ist jedoch ebenfalls zweckmäßig, zusätzlich eines der genannten inerten Lösungsmittel zuzusetzen.

Zur Umsetzung von Hydrazonen der Formel IV können Essigsäure oder ihre reaktionsfähigen Derivate wie z.B. Ester, insbesondere Essigsäuremethyl- oder -ethylester, oder Säurehalogenide, wie z.B. Acetylchlorid oder -bromid, angewendet werden. Man wendet vorteilhaft einen Überschuß von Essigsäure oder ihren Derivaten an, der gleichzeitig als Lösungsmittel dienen kann. Es ist jedoch ebenfalls zweckmäßig, zusätzlich eines der genannten inerten Lösungsmittel zuzusetzen.

Aus z.B. Trialkylphosphiten und entsprechenden Halogenmethylpyridazinonen wie u.a. bei Kosolapoff, Organophosphorus Compounds, John Wiley, New York, Kap. 7, beschriebenen erhaltene Dialkylphosphonate der Formel VI können mit bekannten Aldehyden und Ketonen der Formel V, worin jeweils R bis $R^7$ die bei Formel I angegebene Bedeutung haben, in Anwesenheit einer Base unter den z.B. in Organic Reactions, Bd. 25, John Wiley, New York, 1978, Kap. 2, beschriebenen Bedingungen zu den erfindungsgemäßen Verbindungen der Formel I umgesetzt werden. Als Basen verwendet man beispielsweise Alkalimetallamide wie Kalium- oder Natriumamid oder andere geeignete organische Basen wie Lithiumdiisopropylamid oder Natriummethylsulfinylmethylid. Zweckmäßig führt man die Reaktion in einem inerten Lösungsmittel durch. Als inerte Lösungsmittel eignen sich vorzugsweise Ether wie Diethylether, Ethylenglykoldimethylether, Tetrahydrofuran oder Dioxan sowie Amide wie Dimethylformamid, Hexamethylphosphorsäuretriamid, Dimethylacetamid oder N-Methylpyrrolidon, ferner Sulfoxide wie Dimethylsulfoxid oder Sulfolan sowie Kohlenwasserstoffe wie Pentan,

Hexan, Cyclohexan, Benzol oder Toluol. Die Reaktionstemperaturen liegen zweckmäßig entsprechend der Reaktivität des eingesetzten Phosphonatanions zwischen etwa -10 und etwa 150 °C, vorzugsweise zwischen 20 und 100 °C, die Reaktionszeiten zwischen etwa 1 und 48 Stunden.

Sulfoxide und Sulfone der Formel I, worin mindestens einer der die Phenylgruppe $R^1$ substituierenden Reste SOR oder $SO_2R$ bedeutet, können durch Oxydation entsprechender Thioether der Formel I, worin mindestens einer der Substituenten SR bedeutet, hergestellt werden.

Man oxidiert je nach dem gewählten Reagenz und den angewendeten Bedingungen zu den entsprechenden Sulfoxiden (einer der Substituenten bedeutet SO) oder zu den entsprechenden Sulfonen (einer der Substituenten bedeutet $SO_2$), wobei man nach an sich aus der Literatur bekannten Methoden arbeitet und die Reaktionsbedingungen im einzelnen aus der Literatur leicht entnehmen kann. Will man die Sulfoxide erhalten, so oxydiert man beispielsweise mit Wasserstoffperoxid, Persäuren, Ce(IV)-Verbindungen wie $(NH_4)_2Ce(NO_3)_6$ oder elektrolytisch unter verhältnismäßig milden Bedingungen und bei relativ niedrigen Temperaturen (etwa -80 bis +40°). Will man dagegen die Sulfone erhalten, so verwendet man die gleichen Oxydationsmittel unter kräftigeren Bedingungen und/oder im Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässrige Mineralsäuren, wässrige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$.

Ein bevorzugtes Oxidationsmittel ist 30%iges wässeriges Wasserstoffperoxid. Dieses führt bei Anwendung der berechneten Menge in Lösungsmitteln wie Essigsäure, Aceton oder Ethanol bei Temperaturen zwischen -20 und 30° zu den Sulfoxiden, im Überschuß bei höheren Temperaturen, vorzugsweise in Essigsäure oder in einem Gemisch aus Essigsäure und Acetanhydrid, zu den Sulfonen.

Ein weiteres bevorzugtes Oxidationsmittel ist 3-Chlorperbenzoesäure. Diese führt bei Anwendung der berechneten Menge in Lösungsmitteln wie Halogenkohlenwasserstoffen bei Temperaturen unterhalb von 0° zu den Sulfoxiden, im Überschuß bei Temperaturen zwischen 0° und Raumtemperatur zu den Sulfonen.

Eine Base der Formel I kann mit einer Säure in das zugehörige Säureadditionssalz überführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophophorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie ß-Camphersulfonsäure.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischen Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline.

Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I bei der Bekämpfung von Krankheiten, insbesondere der Herzinsuffizienz und der arteriellen Hypertonie sowie ihre Verwendung bei der therapeutischen Behandlung des menschlichen oder tierischen Körpers.

Dabei werden die erfindungsgemäßen Substanzen in der Regel in Analogie zu bekannten positiv inotrop wirksamen Substanzen, wie Amrinon, verabreicht, vorzugsweise in Dosierungen zwischen etwa 5 und 500 mg, insbesondere zwischen 20 und 100 mg pro Dosierungseinheit. Die tägliche Dosierung liegt vorzugsweise zwischen etwa 0,1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

Beispiel 1

4,5 g 3-Methyl-6-(3-methoxy-4-methylthiophenyl)-4-oxo-5-hexensäure (erhalten aus Methyllävulinsäure und 3-Methoxy-4-methylthiobenzaldehyd in Toluol unter Piperidin- und Eisessigzusatz am Wasserabscheider) und 2 g Hydrazinhydrat werden in 70 ml Ethanol 2 Stunden bei 20 °C gerührt. Die Lösung wird eingeengt, in Dichlormethan aufgenommen, mit Sodalösung ausgeschüttelt, getrocknet und eingedampft. Nach Umkristallisation aus Ethanol erhält man 4,5-Dihydro-5-methyl-6-(3-methoxy-4-methylthio-styryl)-pyridazin-3-on; F: 155 - 156 °C.

Analog erhält man durch Umsetzung der entsprechenden 4-Oxo-5-hexensäuren mit Hydrazinhydrat:

4,5-Dihydro-6-(2-methylthio-styryl)-pyridazin-3-on
4,5-Dihydro-6-(3-methylthio-styryl)-pyridazin-3-on
4,5-Dihydro-6-(4-methylthio-styryl)-pyridazin-3-on
4,5-Dihydro-6-(2-methoxy-styryl)-pyridazin-3-on
4,5-Dihydro-6-(3-methoxy-styryl)-pyridazin-3-on, F: 130 - 131 °C
4,5-Dihydro-6-(2,4-dimethoxy-styryl)-pyridazin-3-on, F : 182 °C
4,5-Dihydro-6-(3,4-dimethoxy-styryl)-pyridazin-3-on, F : 155 - 156 °C
4,5-Dihydro-6-(2-allyloxy-styryl)-pyridazin-3-on
4,5-Dihydro-6-(3-allyloxy-styryl)-pyridazin-3-on
4,5-Dihydro-6-(4-allyloxy-styryl)-pyridazin-3-on
4,5-Dihydro-6-(2-allylthio-styryl)-pyridazin-3-on
4,5-Dihydro-6-(3-allylthio-styryl)-pyridazin-3-on
4,5-Dihydro-6-(4-allylthio-styryl)-pyridazin-3-on
4,5-Dihydro-6-(2-propargyloxy-styryl)-pyridazin-3-on
4,5-Dihydro-6-(3-propargyloxy-styryl)-pyridazin-3-on
4,5-Dihydro-6-(4-propargyloxy-styryl)-pyridazin-3-on
4,5-Dihydro-6-(2-propargylthio-styryl)-pyridazin-3-on
4,5-Dihydro-6-(3-propargylthio-styryl)-pyridazin-3-on
4,5-Dihydro-6-(4-propargylthio-styryl)-pyridazin-3-on
4,5-Dihydro-6-(3-allylthio-4-methyl-styryl)-pyridazin-3-on
4,5-Dihydro-6-(4-allylthio-3-methyl-styryl)-pyridazin-3-on

4,5-Dihydro-6-(3-methoxy-4-propargyloxy-styryl)-pyridazin-3-on, F 199 - 201 °C

4,5-Dihydro-6-(2-methoxy-3-methylthio-styryl)-pyridazin-3-on

4,5-Dihydro-6-(2-methoxy-4-methylthio-styryl)-pyridazin-3-on

4,5-Dihydro-6-(2-methoxy-5-methylthio-styryl)-pyridazin-3-on

4,5-Dihydro-6-(2-methoxy-6-methylthio-styryl)-pyridazin-3-on

4,5-Dihydro-6-(3-methoxy-2-methylthio-styryl)-pyridazin-3-on

4,5-Dihydro-6-(3-methoxy-4-methylthio-styryl)-pyridazin-3-on, F 148 - 149 °C

4,5-Dihydro-6-(3-methoxy-5-methylthio-styryl)-pyridazin-3-on

4,5-Dihydro-6-(3-methoxy-6-methylthio-styryl)-pyridazin-3-on

4,5-Dihydro-6-(4-methoxy-2-methylthio-styryl)-pyridazin-3-on

4,5-Dihydro-6-(4-methoxy-3-methylthio-styryl)-pyridazin-3-on

4,5-Dihydro-6-(3,5-dimethoxy-4-methylthio-styryl)-pyridazin-3-on

4,5-Dihydro-6-(3,4-dimethoxy-5-methylthio-styryl)-pyridazin-3-on

4,5-Dihydro-6-(2,5-dimethoxy-4-methylthio-styryl)-pyridazin-3-on

4,5-Dihydro-6-(2,3,5-trimethoxy-4-methylthio-styryl)-pyridazin-3-on

4,5-Dihydro-5-methyl-6-(2-methylthio-styryl)-pyridazin-3-on

4,5-Dihydro-5-methyl-6-(3-methylthio-styryl)-pyridazin-3-on

4,5-Dihydro-5-methyl-6-(4-methylthio-styryl)-pyridazin-3-on, F: 176 °C

4,5-Dihydro-5-methyl-6-(2-methoxy-styryl)-pyridazin-3-on, F: 110 °C

4,5-Dihydro-5-methyl-6-(3-methoxy-styryl)-pyridazin-3-on

4,5-Dihydro-5-methyl-6-(4-methoxy-styryl)-pyridazin-3-on, F: 158 °C

4,5-Dihydro-5-methyl-6-(3-chlor-4-methoxy-styryl)-pyridazin-3-on, F: 165 °C

4,5-Dihydro-5-methyl-6-(2,3-dimethoxy-styryl)-pyridazin-3-on, F: 152 °C

4,5-Dihydro-5-methyl-6-(3,4-dimethoxy-styryl)-pyridazin-3-on, F: 129 - 130 °C

4,5-Dihydro-5-methyl-6-(3,4,5-trimethoxy-styryl)-pyridazin-3-on, F: 178 °C

4,5-Dihydro-5-methyl-6-(2-allyloxy-styryl)-pyridazin-3-on

4,5-Dihydro-5-methyl-6-(3-allyloxy-styryl)-pyridazin-3-on

4,5-Dihydro-5-methyl-6-(4-allyloxy-styryl)-pyridazin-3-on

4,5-Dihydro-5-methyl-6-(2-allylthio-styryl)-pyridazin-3-on

4,5-Dihydro-5-methyl-6-(3-allylthio-styryl)-pyridazin-3-on

4,5-Dihydro-5-methyl-6-(4-allylthio-styryl)-pyridazin-3-on

4,5-Dihydro-5-methyl-6-(2-propargyloxy-styryl)-pyridazin-3-on

4,5-Dihydro-5-methyl-6-(3-propargyloxy-styryl)-pyridazin-3-on

4,5-Dihydro-5-methyl-6-(4-propargyloxy-styryl)-pyridazin-3-on

4,5-Dihydro-5-methyl-6-(2-propargylthio-styryl)-pyridazin-3-on

4,5-Dihydro-5-methyl-6-(3-propargylthio-styryl)-pyridazin-3-on

4,5-Dihydro-5-methyl-6-(4-propargylthio-styryl)-pyridazin-3-on

4,5-Dihydro-5-methyl-6-(3-allylthio-4-methoxy-styryl)-pyridazin-3-on

4,5-Dihydro-5-methyl-6-(4-allylthio-3-methoxy-styryl)-pyridazin-3-on

4,5-Dihydro-5-methyl-6-(3-methoxy-4-propargyloxy-styryl)-pyridazin-3-on, F: 136 °C

4,5-Dihydro-5-methyl-6-(2-methoxy-3-methylthio-styryl)-pyridazin-3-on

4,5-Dihydro-5-methyl-6-(2-methoxy-4-methylthio-styryl)-pyridazin-3-on, F: 140 °C

4,5-Dihydro-5-methyl-6-(2-methoxy-5-methylthio-styryl)-pyridazin-3-on

4,5-Dihydro-5-methyl-6-(2-methoxy-6-methylthio-styryl)-pyridazin-3-on

4,5-Dihydro-5-methyl-6-(3-methoxy-2-methylthio-styryl)-pyridazin-3-on

4,5-Dihydro-5-methyl-6-(3-methoxy-4-methylthio-styryl)-pyridazin-3-on

4,5-Dihydro-5-methyl-6-(3-methoxy-5-methylthio-styryl)-pyridazin-3-on

4,5-Dihydro-5-methyl-6-(3-methoxy-6-methylthio-styryl)-pyridazin-3-on

4,5-Dihydro-5-methyl-6-(4-methoxy-2-methylthio-styryl)-pyridazin-3-on

4,5-Dihydro-5-methyl-6-(4-methoxy-3-methylthio-styryl)-pyridazin-3-on, F: 162 °C

4,5-Dihydro-5-methyl-6-(3,5-dimethoxy-4-methylthio-styryl)-pyridazin-3-on

4,5-Dihydro-5-methyl-6-(3,4-dimethoxy-5-methylthio-styryl)-pyridazin-3-on

4,5-Dihydro-5-methyl-6-(2,5-dimethoxy-4-methylthio-styryl)-pyridazin-3-on

4,5-Dihydro-5-methyl-6-(2,3,5-trimethoxy-4-methyl-thio-styryl)-pyridazin-3-on

4,5-Dihydro-5-ethyl-6-(4-methoxy-styryl)-pyridazin-3-on, F: 146 °C

4,5-Dihydro-5-ethyl-6-(3-methoxy-4-methylthio-styryl)-pyridazin-3-on, F: 160-162 °C

4,5-Dihydro-5-methyl-6-[2-(2-methylthiophenyl)-prop-1-enyl]-pyridazin-3-on
4,5-Dihydro-5-methyl-6-[2-(3-methylthiophenyl)-prop-1-enyl]-pyridazin-3-on
4,5-Dihydro-5-methyl-6-[2-(4-methylthiophenyl)-prop-1-enyl]-pyridazin-3-on
4,5-Dihydro-5-methyl-6-[2-(2-methoxyphenyl)-prop-1-enyl]-pyridazin-3-on
4,5-Dihydro-5-methyl-6-[2-(3-methoxyphenyl)-prop-1-enyl]-pyridazin-3-on
4,5-Dihydro-5-methyl-6-[2-(4-methoxyphenyl)-prop-1-enyl]-pyridazin-3-on
4,5-Dihydro-5-methyl-6-[2-(2-allyloxyphenyl)-prop-1-enyl]-pyridazin-3-on
4,5-Dihydro-5-methyl-6-[2-(3-allyloxyphenyl)-prop-1-enyl]-pyridazin-3-on
4,5-Dihydro-5-methyl-6-[2-(4-allyloxyphenyl)-prop-1-enyl]-pyridazin-3-on
4,5-Dihydro-5-methyl-6-[2-(2-allylthiophenyl)-prop-1-enyl]-pyridazin-3-on
4,5-Dihydro-5-methyl-6-[2-(3-allylthiophenyl)-prop-1-enyl]-pyridazin-3-on
4,5-Dihydro-5-methyl-6-[2-(4-allylthiophenyl)-prop-1-enyl]-pyridazin-3-on
4,5-Dihydro-5-methyl-6-[2-(2-propargyloxyphenyl)-prop-1-enyl]-pyridazin-3-on
4,5-Dihydro-5-methyl-6-[2-(3-propargyloxyphenyl)-prop-1-enyl]-pyridazin-3-on
4,5-Dihydro-5-methyl-6-[2-(4-propargyloxyphenyl)-prop-1-enyl]-pyridazin-3-on
4,5-Dihydro-5-methyl-6-[2-(2-propargylthiophenyl)-prop-1-enyl]-pyridazin-3-on
4,5-Dihydro-5-methyl-6-[2-(3-propargylthiophenyl)-prop-1-enyl]-pyridazin-3-on
4,5-Dihydro-5-methyl-6-[2-(4-propargylthiophenyl)-prop-1-enyl]-pyridazin-3-on

4,5-Dihydro-5-methyl-6-[2-(3-allylthio-4-methoxyphenyl)-prop-1-enyl]-pyridazin-3-on

4,5-Dihydro-5-methyl-6-[2-(4-allylthio-3-methoxyphenyl)-prop-1-enyl]-pyridazin-3-on

4,5-Dihydro-5-methyl-6-[2-(2-methoxy-3-methylthiophenyl)-prop-1-enyl]-pyridazin-3-on

4,5-Dihydro-5-methyl-6-[2-(2-methoxy-4-methylthiophenyl)-prop-1-enyl]-pyridazin-3-on

4,5-Dihydro-5-methyl-6-[2-(2-methoxy-5-methylthiophenyl)-prop-1-enyl]-pyridazin-3-on

4,5-Dihydro-5-methyl-6-[2-(2-methoxy-6-methylthiophenyl)-prop-1-enyl]-pyridazin-3-on

4,5-Dihydro-5-methyl-6-[2-(3-methoxy-2-methylthiophenyl)-prop-1-enyl]-pyridazin-3-on

4,5-Dihydro-5-methyl-6-[2-(3-methoxy-4-methylthiophenyl)-prop-1-enyl]-pyridazin-3-on

4,5-Dihydro-5-methyl-6-[2-(3-methoxy-5-methylthiophenyl)-prop-1-enyl]-pyridazin-3-on

4,5-Dihydro-5-methyl-6-[2-(3-methoxy-6-methylthiophenyl)-prop-1-enyl]-pyridazin-3-on

4,5-Dihydro-5-methyl-6-[2-(4-methoxy-2-methylthiophenyl)-prop-1-enyl]-pyridazin-3-on

4,5-Dihydro-5-methyl-6-[2-(4-methoxy-3-methylthiophenyl)-prop-1-enyl]-pyridazin-3-on

4,5-Dihydro-5-methyl-6-[2-(3,5-dimethoxy-4-methylthiophenyl)-prop-1-enyl]-pyridazin-3-on

4,5-Dihydro-5-methyl-6-[2-(3,4-dimethoxy-5-methylthiophenyl)-prop-1-enyl]-pyridazin-3-on

4,5-Dihydro-5-methyl-6-[2-(2,5-dimethoxy-4-methylthiophenyl)-prop-1-enyl]-pyridazin-3-on

4,5-Dihydro-5-methyl-6-[2-(2,3,5-trimethoxy-4-methylthiophenyl)-prop-1-enyl]-pyridazin-3-on

4,5-Dihydro-5-methyl-6-[1-methyl-2-(2-methylthiophenyl)-ethenyl]-pyridazin-3-on

4,5-Dihydro-5-methyl-6-[1-methyl-2-(3-methylthiophenyl)-ethenyl]-pyridazin-3-on
4,5-Dihydro-5-methyl-6-[1-methyl-2-(4-methylthiophenyl)-ethenyl]-pyridazin-3-on
4,5-Dihydro-5-methyl-6-[1-methyl-2-(2-methoxyphenyl)-ethenyl]-pyridazin-3-on
4,5-Dihydro-5-methyl-6-[1-methyl-2-(3-methoxyphenyl)-ethenyl]-pyridazin-3-on
4,5-Dihydro-5-methyl-6-[1-methyl-2-(4-methoxyphenyl)-ethenyl]-pyridazin-3-on
4,5-Dihydro-5-methyl-6-[1-methyl-2-(2-allyloxyphenyl)-ethenyl]-pyridazin-3-on
4,5-Dihydro-5-methyl-6-[1-methyl-2-(3-allyloxyphenyl)-ethenyl]-pyridazin-3-on
4,5-Dihydro-5-methyl-6-[1-methyl-2-(4-allyloxyphenyl)-ethenyl]-pyridazin-3-on
4,5-Dihydro-5-methyl-6-[1-methyl-2-(2-allylthiophenyl)-ethenyl]-pyridazin-3-on
4,5-Dihydro-5-methyl-6-[1-methyl-2-(3-allylthiophenyl)-ethenyl]-pyridazin-3-on
4,5-Dihydro-5-methyl-6-[1-methyl-2-(4-allylthiophenyl)-ethenyl]-pyridazin-3-on
4,5-Dihydro-5-methyl-6-[1-methyl-2-(2-propargyloxy-phenyl)-ethenyl]-pyridazin-3-on
4,5-Dihydro-5-methyl-6-[1-methyl-2-(3-propargyloxy-phenyl)-ethenyl]-pyridazin-3-on
4,5-Dihydro-5-methyl-6-[1-methyl-2-(4-propargyloxy-phenyl)-ethenyl]-pyridazin-3-on
4,5-Dihydro-5-methyl-6-[1-methyl-2-(2-propargylthio-phenyl)-ethenyl]-pyridazin-3-on
4,5-Dihydro-5-methyl-6-[1-methyl-2-(3-propargylthio-phenyl)-ethenyl]-pyridazin-3-on
4,5-Dihydro-5-methyl-6-[1-methyl-2-(4-propargylthio-phenyl)-ethenyl]-pyridazin-3-on
4,5-Dihydro-5-methyl-6-[1-methyl-2-(3-allylthio-4-methoxyphenyl)-ethenyl]-pyridazin-3-on

4,5-Dihydro-5-methyl-6-[1-methyl-2-(4-allylthio-3-methoxyphenyl)-ethenyl]-pyridazin-3-on
4,5-Dihydro-5-methyl-6-[1-methyl-2-(3-propargylthio-4-methoxyphenyl)-ethenyl]-pyridazin-3-on
4,5-Dihydro-5-methyl-6-[1-methyl-2-(4-propargylthio-3-methoxyphenyl)-ethenyl]-pyridazin-3-on

4,5-Dihydro-5-methyl-6-[1-methyl-2-(2-methoxy-3-methylthiophenyl)-ethenyl]-pyridazin-3-on
4,5-Dihydro-5-methyl-6-[1-methyl-2-(2-methoxy-4-methylthiophenyl)-ethenyl]-pyridazin-3-on
4,5-Dihydro-5-methyl-6-[1-methyl-2-(2-methoxy-5-methylthiophenyl)-ethenyl]-pyridazin-3-on
4,5-Dihydro-5-methyl-6-[1-methyl-2-(2-methoxy-6-methylthiophenyl)-ethenyl]-pyridazin-3-on
4,5-Dihydro-5-methyl-6-[1-methyl-2-(3-methoxy-2-methylthiophenyl)-ethenyl]-pyridazin-3-on
4,5-Dihydro-5-methyl-6-[1-methyl-2-(3-methoxy-4-methylthiophenyl)-ethenyl]-pyridazin-3-on
4,5-Dihydro-5-methyl-6-[1-methyl-2-(3-methoxy-5-methylthiophenyl)-ethenyl]-pyridazin-3-on
4,5-Dihydro-5-methyl-6-[1-methyl-2-(3-methoxy-6-methylthiophenyl)-ethenyl]-pyridazin-3-on
4,5-Dihydro-5-methyl-6-[1-methyl-2-(4-methoxy-2-methylthiophenyl)-ethenyl]-pyridazin-3-on
4,5-Dihydro-5-methyl-6-[1-methyl-2-(4-methoxy-3-methylthiophenyl)-ethenyl]-pyridazin-3-on
4,5-Dihydro-5-methyl-6-[1-methyl-2-(3,5-dimethoxy-4-methylthiophenyl)-ethenyl]-pyridazin-3-on
4,5-Dihydro-5-methyl-6-[1-methyl-2-(3,4-dimethoxy-5-methylthiophenyl)-ethenyl]-pyridazin-3-on
4,5-Dihydro-5-methyl-6-[1-methyl-2-(2,5-dimethoxy-4-methylthiophenyl)-ethenyl]-pyridazin-3-on
4,5-Dihydro-5-methyl-6-[1-methyl-2-(2,3,5-trimethoxy-4-methylthiophenyl)-ethenyl]-pyridazin-3-on.

Beispiel 2

Zu der Lösung von 11,2 g Semicarbazidhydrochlorid in 300 ml Wasser gibt man 26,4 g 3-Methyl-6-(4-methylthiophenyl)-4-oxo-5-hexensäure (hergestellt aus Methyllävulinsäure und 4-Methylthiobenzaldehyd wie in Beispiel 1 beschrieben) und 100 ml 1N-Natronlauge. Es wird 6 Stunden bei 75 °C gerührt und anschließend durch Zugabe verdünnter Salzsäure angesäuert. Das ausgefallene Semicarbazon wird abfiltriert und in 250 ml Sulfolan gelöst. Dreistündiges Erwärmen auf 120 °C bewirkt den Ringschluß. Nach Gießen des Reaktionsgemisches auf 1 Liter Wasser kann 4,5-Dihydro-5-methyl-6-(4-methylthio-styryl)-pyridazin-3-on abfiltriert werden, F: 176 °C.

Analog erhält man durch Umsetzung der entsprechenden 4-Oxo-hex-5-ensäuren mit Semicarbazidhydrochlorid:

4,5-Dihydro-5-methyl-6-(2-hydroxy-styryl)-pyridazin-3-on
4,5-Dihydro-5-methyl-6-(3-hydroxy-styryl)-pyridazin-3-on
4,5-Dihydro-5-methyl-6-(4-hydroxy-styryl)-pyridazin-3-on
4,5-Dihydro-5-methyl-6-(3-hydroxy-4-methoxy-styryl)-pyridazin-3-on
4,5-Dihydro-5-methyl-6-(4-hydroxy-3-methoxy-styryl)-pyridazin-3-on
4,5-Dihydro-5-methyl-6-(3-hydroxy-4-methylthio-styryl)-pyridazin-3-on
4,5-Dihydro-5-methyl-6-(3-hydroxy-4-methylsulfinyl-styryl)-pyridazin-3-on
4,5-Dihydro-5-methyl-6-(3-hydroxy-4-methylsulfonyl-styryl)-pyridazin-3-on
4,5-Dihydro-5-methyl-6-(2-mercapto-styryl)-pyridazin-3-on
4,5-Dihydro-5-methyl-6-(3-mercapto-styryl)-pyridazin-3-on
4,5-Dihydro-5-methyl-6-(4-mercapto-styryl)-pyridazin-3-on
4,5-Dihydro-5-methyl-6-(2-amino-styryl)-pyridazin-3-on

4,5-Dihydro-5-methyl-6-(3-amino-styryl)-pyridazin-3-on
4,5-Dihydro-5-methyl-6-(4-amino-styryl)-pyridazin-3-on
4,5-Dihydro-6-(2-hydroxy-styryl)-pyridazin-3-on
4,5-Dihydro-6-(3-hydroxy-styryl)-pyridazin-3-on
4,5-Dihydro-6-(4-hydroxy-styryl)-pyridazin-3-on
4,5-Dihydro-6-(3-hydroxy-4-methoxy-styryl)-pyridazin-3-on
4,5-Dihydro-6-(4-hydroxy-3-methoxy-styryl)-pyridazin-3-on
4,5-Dihydro-6-(3-hydroxy-4-methylthio-styryl)-pyridazin-3-on
4,5-Dihydro-6-(3-hydroxy-4-methylsulfinyl-styryl)-pyridazin-3-on
4,5-Dihydro-6-(3-hydroxy-4-methylsulfonyl-styryl)-pyridazin-3-on
4,5-Dihydro-6-(2-mercapto-styryl)-pyridazin-3-on
4,5-Dihydro-6-(3-mercapto-styryl)-pyridazin-3-on
4,5-Dihydro-6-(4-mercapto-styryl)-pyridazin-3-on
4,5-Dihydro-6-(2-amino-styryl)-pyridazin-3-on
4,5-Dihydro-6-(3-amino-styryl)-pyridazin-3-on
4,5-Dihydro-6-(4-amino-styryl)-pyridazin-3-on

Beispiel 3

Zu der Suspension von 3,9 g Natriumamid in 280 ml trockenem Tetrahydrofuran tropft man bei Raumtemperatur die Lösung von 23,5 g 4,5-Dihydropyridazin-3-on-6-yl-methyl)-dimethylphosphonat (hergestellt aus 4,5-Dihydropyridazin-3-on-6-carbonsäuremethylester durch Reduktion mit Lithiumborhydrid zu 4,5-Dihydro-6-hydroxymethylpyridazin-3-on, Umsetzung mit Phosphortribromid zu 6-Brommethyl-4,5-dihydropyridazin-3-on und dessen Reaktion mit Trimethylphosphit) in 80 ml des gleichen Lösungsmittels. Nach 30 Minuten Rühren setzt man 14,1 g 4-Chlorbenzaldehyd zu und kocht noch 5 Stunden.

Anschließend wird vom Lösungsmittel befreit. Man digeriert den Rückstand mit Wasser, filtriert und kristallisiert aus Ethanol um. So wird 4,5-Dihydro-6-(4-chlor-styryl)-pyridazin-3-on erhalten; F: 237 - 238 °C.

Analog erhält man durch Umsetzung der entsprechenden Phosphonate der Formel VI mit den Oxoverbindungen der Formel V:

4,5-Dihydro-6-(4-methylamino-styryl)-pyridazin-3-on
4,5-Dihydro-6-(4-dimethylamino-styryl)-pyridazin-3-on, F: 210 - 212 °C
4,5-Dihydro-6-(4-acetamido-styryl)-pyridazin-3-on
4,5-Dihydro-6-(2-fluor-styryl)-pyridazin-3-on
4,5-Dihydro-6-(3-fluor-styryl)-pyridazin-3-on
4,5-Dihydro-6-(4-fluor-styryl)-pyridazin-3-on
4,5-Dihydro-6-(4-brom-styryl)-pyridazin-3-on
4,5-Dihydro-6-(4-jod-styryl)-pyridazin-3-on
4,5-Dihydro-6-(2-trifluormethyl-styryl)-pyridazin-3-on
4,5-Dihydro-6-(3-trifluormethyl-styryl)-pyridazin-3-on
4,5-Dihydro-6-(4-trifluormethyl-styryl)-pyridazin-3-on
4,5-Dihydro-6-(4-nitro-styryl)-pyridazin-3-on
4,5-Dihydro-6-(4-cyan-styryl)-pyridazin-3-on
4,5-Dihydro-6-(2-carbamoyl-styryl)-pyridazin-3-on
4,5-Dihydro-6-(4-carbamoyl-styryl)-pyridazin-3-on
4,5-Dihydro-5-methyl-6-(3-methyl-4-methoxy-styryl)-pyridazin-3-on, F: 133 - 134 °C
4,5-Dihydro-5-methyl-6-(2-chlor-4-methylthio-styryl)-pyridazin-3-on, F: 145 - 147 °C
4,5-Dihydro-5-methyl-6-(4-methylamino-styryl)-pyridazin-3-on
4,5-Dihydro-5-methyl-6-(4-dimethylamino-styryl)-pyridazin-3-on
4,5-Dihydro-5-methyl-6-(4-acetamido-styryl)-pyridazin-3-on, F: 210 °C

4,5-Dihydro-5-methyl-6-(2-fluor-styryl)-pyridazin-3-on
4,5-Dihydro-5-methyl-6-(3-fluor-styryl)-pyridazin-3-on
4,5-Dihydro-5-methyl-6-(4-fluor-styryl)-pyridazin-3-on, F: 168 °C
4,5-Dihydro-5-methyl-6-(4-chlor-styryl)-pyridazin-3-on, F: 175 °C
4,5-Dihydro-5-methyl-6-(4-brom-styryl)-pyridazin-3-on
4,5-Dihydro-5-methyl-6-(4-jod-styryl)-pyridazin-3-on
4,5-Dihydro-5-methyl-6-(2-trifluormethyl-styryl)-pyridazin-3-on
4,5-Dihydro-5-methyl-6-(3-trifluormethyl-styryl)-pyridazin-3-on
4,5-Dihydro-5-methyl-6-(4-trifluormethyl-styryl)-pyridazin-3-on
4,5-Dihydro-5-methyl-6-(4-nitro-styryl)-pyridazin-3-on
4,5-Dihydro-5-methyl-6-(4-cyan-styryl)-pyridazin-3-on
4,5-Dihydro-5-methyl-6-(2-carbamoyl-styryl)-pyridazin-3-on
4,5-Dihydro-5-methyl-6-(4-carbamoyl-styryl)-pyridazin-3-on

Beispiel 4

29 g nach Beispiel 1 erhaltenes 4,5-Dihydro-5-methyl-6-(3-methoxy-4-methylthio-styryl)-pyridazin-3-on werden in 300 ml Eisessig gelöst und mit 6,7 g 30 %igem Wasserstoffperoxid versetzt. Nach 10 Stunden Rühren bei Raumtemperatur filtriert man das ausgefallene 4,5-Dihydro-5-methyl-6-(3-methoxy-4-methylsulfinyl-styryl)-pyridazin-3-on ab; F: 175 - 176 °C.

Analog erhält man durch Umsetzung der entsprechenden Thioether mit Wasserstoffperoxid

6-(2-Methylsulfinyl-styryl)-pyridazin-3-on
6-(3-Methylsulfinyl-styryl)-pyridazin-3-on
6-(4-Methylsulfinyl-styryl)-pyridazin-3-on

6-(2-Allylsulfinyl-styryl)-pyridazin-3-on
6-(3-Allylsulfinyl-styryl)-pyridazin-3-on
6-(4-Allylsulfinyl-styryl)-pyridazin-3-on
6-(2-Propargylsulfinyl-styryl)-pyridazin-3-on
6-(3-Propargylsulfinyl-styryl)-pyridazin-3-on
6-(4-Propargylsulfinyl-styryl)-pyridazin-3-on
6-(3-Allylsulfinyl-4-methoxy-styryl)-pyridazin-3-on
6-(4-Allylsulfinyl-3-methoxy-styryl)-pyridazin-3-on
6-(3-Propargylsulfinyl-4-methoxy-styryl)-pyridazin-3-on
6-(4-Propargylsulfinyl-3-methoxy-styryl)-pyridazin-3-on
6-(2-Methoxy-3-methylsulfinyl-styryl)-pyridazin-3-on
6-(2-Methoxy-4-methylsulfinyl-styryl)-pyridazin-3-on
6-(2-Methoxy-5-methylsulfinyl-styryl)-pyridazin-3-on
6-(2-Methoxy-6-methylsulfinyl-styryl)-pyridazin-3-on
6-(3-Methoxy-2-methylsulfinyl-styryl)-pyridazin-3-on
6-(3-Methoxy-4-methylsulfinyl-styryl)-pyridazin-3-on
6-(3-Methoxy-5-methylsulfinyl-styryl)-pyridazin-3-on
6-(3-Methoxy-6-methylsulfinyl-styryl)-pyridazin-3-on
6-(4-Methoxy-2-methylsulfinyl-styryl)-pyridazin-3-on
6-(4-Methoxy-3-methylsulfinyl-styryl)-pyridazin-3-on
6-(3,5-Dimethoxy-4-methylsulfinyl-styryl)-pyridazin-3-on
6-(3,4-Dimethoxy-5-methylsulfinyl-styryl)-pyridazin-3-on
6-(2,5-Dimethoxy-4-methylsulfinyl-styryl)-pyridazin-3-on
6-(2,3,5-Trimethoxy-4-methylsulfinyl-styryl)-pyridazin-3-on

4,5-Dihydro-6-(2-methylsulfinyl-styryl)-pyridazin-3-on
4,5-Dihydro-6-(3-methylsulfinyl-styryl)-pyridazin-3-on
4,5-Dihydro-6-(4-methylsulfinyl-styryl)-pyridazin-3-on
4,5-Dihydro-6-(2-allylsulfinyl-styryl)-pyridazin-3-on
4,5-Dihydro-6-(3-allylsulfinyl-styryl)-pyridazin-3-on
4,5-Dihydro-6-(4-allylsulfinyl-styryl)-pyridazin-3-on
4,5-Dihydro-6-(2-propargylsulfinyl-styryl)-pyridazin-3-on
4,5-Dihydro-6-(3-propargylsulfinyl-styryl)-pyridazin-3-on
4,5-Dihydro-6-(4-propargylsulfinyl-styryl)-pyridazin-3-on

4,5-Dihydro-6-(3-allylsulfinyl-4-methoxy-styryl)-pyridazin-3-on

4,5-Dihydro-6-(4-allylsulfinyl-3-methoxy-styryl)-pyridazin-3-on

4,5-Dihydro-6-(3-propargylsulfinyl-4-methoxy-styryl)-pyridazin-3-on

4,5-Dihydro-6-(4-propargylsulfinyl-3-methoxy-styryl)-pyridazin-3-on

4,5-Dihydro-6-(2-methoxy-3-methylsulfinyl-styryl)-pyridazin-3-on

4,5-Dihydro-6-(2-methoxy-4-methylsulfinyl-styryl)-pyridazin-3-on

4,5-Dihydro-6-(2-methoxy-5-methylsulfinyl-styryl)-pyridazin-3-on

4,5-Dihydro-6-(2-methoxy-6-methylsulfinyl-styryl)-pyridazin-3-on

4,5-Dihydro-6-(3-methoxy-2-methylsulfinyl-styryl)-pyridazin-3-on

4,5-Dihydro-6-(3-methoxy-4-methylsulfinyl-styryl)-pyridazin-3-on

4,5-Dihydro-6-(3-methoxy-5-methylsulfinyl-styryl)-pyridazin-3-on

4,5-Dihydro-6-(3-methoxy-6-methylsulfinyl-styryl)-pyridazin-3-on

4,5-Dihydro-6-(4-methoxy-2-methylsulfinyl-styryl)-pyridazin-3-on

4,5-Dihydro-6-(4-methoxy-3-methylsulfinyl-styryl)-pyridazin-3-on

4,5-Dihydro-6-(3,5-dimethoxy-4-methylsulfinyl-styryl)-pyridazin-3-on

4,5-Dihydro-6-(3,4-dimethoxy-5-methylsulfinyl-styryl)-pyridazin-3-on

4,5-Dihydro-6-(2,5-dimethoxy-4-methylsulfinyl-styryl)-pyridazin-3-on

4,5-Dihydro-6-(2,3,5-trimethoxy-4-methylsulfinyl--styryl)-pyridazin-3-on

4,5-Dihydro-5-methyl-6-(2-methylsulfinyl-styryl)-pyridazin-3-on
4,5-Dihydro-5-methyl-6-(3-methylsulfinyl-styryl)-pyridazin-3-on
4,5-Dihydro-5-methyl-6-(4-methylsulfinyl-styryl)-pyridazin-3-on, F: 178 °C
4,5-Dihydro-5-methyl-6-(2-allylsulfinyl-styryl)-pyridazin-3-on
4,5-Dihydro-5-methyl-6-(3-allylsulfinyl-styryl)-pyridazin-3-on
4,5-Dihydro-5-methyl-6-(4-allylsulfinyl-styryl)-pyridazin-3-on
4,5-Dihydro-5-methyl-6-(2-propargylsulfinyl-styryl)-pyridazin-3-on
4,5-Dihydro-5-methyl-6-(3-propargylsulfinyl-styryl)-pyridazin-3-on
4,5-Dihydro-5-methyl-6-(4-propargylsulfinyl-styryl)-pyridazin-3-on
4,5-Dihydro-5-methyl-6-(3-allylsulfinyl-4-methoxy-styryl)-pyridazin-3-on
4,5-Dihydro-5-methyl-6-(4-allylsulfinyl-3-methoxy-styryl)-pyridazin-3-on
4,5-Dihydro-5-methyl-6-(3-propargylsulfinyl-4-methoxy-styryl)-pyridazin-3-on
4,5-Dihydro-5-methyl-6-(4-propargylsulfinyl-3-methoxy-styryl)-pyridazin-3-on
4,5-Dihydro-5-methyl-6-(2-methoxy-3-methylsulfinyl--styryl)-pyridazin-3-on
4,5-Dihydro-5-methyl-6-(2-methoxy-4-methylsulfinyl--styryl)-pyridazin-3-on
4,5-Dihydro-5-methyl-6-(2-methoxy-5-methylsulfinyl--styryl)-pyridazin-3-on
4,5-Dihydro-5-methyl-6-(2-methoxy-6-methylsulfinyl--styryl)-pyridazin-3-on
4,5-Dihydro-5-methyl-6-(3-methoxy-2-methylsulfinyl--styryl)-pyridazin-3-on

4,5-Dihydro-5-methyl-6-(3-methoxy-5-methylsulfinyl-styryl)-pyridazin-3-on

4,5-Dihydro-5-methyl-6-(3-methoxy-4-methylsulfinyl-styryl)-pyridazin-3-on, F: 175 °C

4,5-Dihydro-5-methyl-6-(4-methoxy-2-methylsulfinyl-styryl)-pyridazin-3-on

4,5-Dihydro-5-methyl-6-(4-methoxy-3-methylsulfinyl-styryl)-pyridazin-3-on, F: 205 °C

4,5-Dihydro-5-methyl-6-(3,5-dimethoxy-4-methylsulfinyl-styryl)-pyridazin-3-on

4,5-Dihydro-5-methyl-6-(3,4-dimethoxy-5-methylsulfinyl-styryl)-pyridazin-3-on

4,5-Dihydro-5-methyl-6-(2,5-dimethoxy-4-methylsulfinyl-styryl)-pyridazin-3-on

4,5-Dihydro-5-methyl-6-(2,3,5-trimethoxy-4-methylsulfinyl-styryl)-pyridazin-3-on

4,5-Dihydro-5-methyl-6-(2-chlor-4-methylsulfinyl-styryl)-pyridazin-3-on, F: 226-228 °C

4,5-Dihydro-5-methyl-6-[2-(2-methylsulfinylphenyl)-prop-1-enyl]-pyridazin-3-on

4,5-Dihydro-5-methyl-6-[2-(3-methylsulfinylphenyl)-prop-1-enyl]-pyridazin-3-on

4,5-Dihydro-5-methyl-6-[2-(4-methylsulfinylphenyl)-prop-1-enyl]-pyridazin-3-on

4,5-Dihydro-5-methyl-6-[2-(2-allylsulfinylphenyl)-prop-1-enyl]-pyridazin-3-on

4,5-Dihydro-5-methyl-6-[2-(3-allylsulfinylphenyl)-prop-1-enyl]-pyridazin-3-on

4,5-Dihydro-5-methyl-6-[2-(4-allylsulfinylphenyl)-prop-1-enyl]-pyridazin-3-on

4,5-Dihydro-5-methyl-6-[2-(2-propargylsulfinylphenyl)-prop-1-enyl]-pyridazin-3-on

4,5-Dihydro-5-methyl-6-[2-(3-propargylsulfinylphenyl)-prop-1-enyl]-pyridazin-3-on

4,5-Dihydro-5-methyl-6-[2-(4-propargylsulfinylphenyl)-prop-1-enyl]-pyridazin-3-on
4,5-Dihydro-5-methyl-6-[2-(3-allylsulfinyl-4-methoxyphenyl)-prop-1-enyl]-pyridazin-3-on
4,5-Dihydro-5-methyl-6-[2-(4-allylsulfinyl-3-methoxyphenyl)-prop-1-enyl]-pyridazin-3-on
4,5-Dihydro-5-methyl-6-[2-(3-propargylsulfinyl-4-methoxyphenyl)-prop-1-enyl]-pyridazin-3-on
4,5-Dihydro-5-methyl-6-[2-(4-propargylsulfinyl-3-methoxyphenyl)-prop-1-enyl]-pyridazin-3-on
4,5-Dihydro-5-methyl-6-[2-(2-methoxy-3-methylsulfinylphenyl)-prop-1-enyl]-pyridazin-3-on
4,5-Dihydro-5-methyl-6-[2-(2-methoxy-4-methylsulfinylphenyl)-prop-1-enyl]-pyridazin-3-on
4,5-Dihydro-5-methyl-6-[2-(2-methoxy-5-methylsulfinylphenyl)-prop-1-enyl]-pyridazin-3-on
4,5-Dihydro-5-methyl-6-[2-(2-methoxy-6-methylsulfinylphenyl)-prop-1-enyl]-pyridazin-3-on
4,5-Dihydro-5-methyl-6-[2-(3-methoxy-2-methylsulfinylphenyl)-prop-1-enyl]-pyridazin-3-on
4,5-Dihydro-5-methyl-6-[2-(3-methoxy-4-methylsulfinylphenyl)-prop-1-enyl]-pyridazin-3-on
4,5-Dihydro-5-methyl-6-[2-(3-methoxy-5-methylsulfinylphenyl)-prop-1-enyl]-pyridazin-3-on
4,5-Dihydro-5-methyl-6-[2-(3-methoxy-6-methylsulfinylphenyl)-prop-1-enyl]-pyridazin-3-on
4,5-Dihydro-5-methyl-6-[2-(4-methoxy-2-methylsulfinylphenyl)-prop-1-enyl]-pyridazin-3-on
4,5-Dihydro-5-methyl-6-[2-(4-methoxy-3-methylsulfinylphenyl)-prop-1-enyl]-pyridazin-3-on
4,5-Dihydro-5-methyl-6-[2-(3,5-dimethoxy-4-methylsulfinylphenyl)-prop-1-enyl]-pyridazin-3-on
4,5-Dihydro-5-methyl-6-[2-(3,4-dimethoxy-5-methylsulfinylphenyl)-prop-1-enyl]-pyridazin-3-on
4,5-Dihydro-5-methyl-6-[2-(2,5-dimethoxy-4-methylsulfinylphenyl)-prop-1-enyl]-pyridazin-3-on

4,5-Dihydro-5-methyl-6-[2-(2,3,5-trimethoxy-3-methylsulfinylphenyl)-prop-1-enyl]-pyridazin-3-on

4,5-Dihydro-5-methyl-6-[1-methyl-2-(2-methylsulfinylphenyl)-ethenyl]-pyridazin-3-on
4,5-Dihydro-5-methyl-6-[1-methyl-2-(3-methylsulfinylphenyl)-ethenyl]-pyridazin-3-on
4,5-Dihydro-5-methyl-6-[1-methyl-2-(4-methylsulfinylphenyl)-ethenyl]-pyridazin-3-on
4,5-Dihydro-5-methyl-6-[1-methyl-2-(2-allylsulfinylphenyl)-ethenyl]-pyridazin-3-on
4,5-Dihydro-5-methyl-6-[1-methyl-2-(3-allylsulfinylphenyl)-ethenyl]-pyridazin-3-on
4,5-Dihydro-5-methyl-6-[1-methyl-2-(4-allylsulfinylphenyl)-ethenyl]-pyridazin-3-on
4,5-Dihydro-5-methyl-6-[1-methyl-2-(2-propargylsulfinylphenyl)-ethenyl]-pyridazin-3-on
4,5-Dihydro-5-methyl-6-[1-methyl-2-(3-propargylsulfinylphenyl)-ethenyl]-pyridazin-3-on
4,5-Dihydro-5-methyl-6-[1-methyl-2-(4-propargylsulfinylphenyl)-ethenyl]-pyridazin-3-on
4,5-Dihydro-5-methyl-6-[1-methyl-2-(3-allylsulfinyl-4-methoxyphenyl)-ethenyl]-pyridazin-3-on
4,5-Dihydro-5-methyl-6-[1-methyl-2-(4-allylsulfinyl-4-methoxyphenyl)-ethenyl]-pyridazin-3-on
4,5-Dihydro-5-methyl-6-[1-methyl-2-(3-propargylsulfinyl-4-methoxyphenyl)-ethenyl]-pyridazin-3-on
4,5-Dihydro-5-methyl-6-[1-methyl-2-(4-propargylsulfinyl-4-methoxyphenyl)-ethenyl]-pyridazin-3-on

4,5-Dihydro-5-methyl-6-[1-methyl-2-(2-methoxy-3-methylsulfinylphenyl)-ethenyl]-pyridazin-3-on
4,5-Dihydro-5-methyl-6-[1-methyl-2-(2-methoxy-4-methylsulfinylphenyl)-ethenyl]-pyridazin-3-on
4,5-Dihydro-5-methyl-6-[1-methyl-2-(2-methoxy-5-methylsulfinylphenyl)-ethenyl]-pyridazin-3-on

4,5-Dihydro-5-methyl-6-[1-methyl-2-(2-methoxy-6-methylsulfinylphenyl)-ethenyl]-pyridazin-3-on
4,5-Dihydro-5-methyl-6-[1-methyl-2-(3-methoxy-2-methylsulfinylphenyl)-ethenyl]-pyridazin-3-on
4,5-Dihydro-5-methyl-6-[1-methyl-2-(3-methoxy-4-methylsulfinylphenyl)-ethenyl]-pyridazin-3-on
4,5-Dihydro-5-methyl-6-[1-methyl-2-(3-methoxy-5-methylsulfinylphenyl)-ethenyl]-pyridazin-3-on
4,5-Dihydro-5-methyl-6-[1-methyl-2-(3-methoxy-6-methylsulfinylphenyl)-ethenyl]-pyridazin-3-on
4,5-Dihydro-5-methyl-6-[1-methyl-2-(4-methoxy-2-methylsulfinylphenyl)-ethenyl]-pyridazin-3-on
4,5-Dihydro-5-methyl-6-[1-methyl-2-(4-methoxy-3-methylsulfinylphenyl)-ethenyl]-pyridazin-3-on
4,5-Dihydro-5-methyl-6-[1-methyl-2-(3,5-dimethoxy-4-methylsulfinylphenyl)-ethenyl]-pyridazin-3-on
4,5-Dihydro-5-methyl-6-[1-methyl-2-(3,4-dimethoxy-5-methylsulfinylphenyl)-ethenyl]-pyridazin-3-on
4,5-Dihydro-5-methyl-6-[1-methyl-2-(2,5-dimethoxy-4-methylsulfinylphenyl)-ethenyl]-pyridazin-3-on
4,5-Dihydro-5-methyl-6-[1-methyl-2-(2,3,5-trimethoxy-3-methylsulfinylphenyl)-ethenyl]-pyridazin-3-on

Beispiel 5

29 g nach Beispiel 1 erhaltenes 4,5-Dihydro-5-methyl-6-(3-methylthio-4-methoxy-styryl)-pyridazin-3-on werden in 250 ml Eisessig gelöst, mit 10 g 85 %igem Wasserstoffperoxid versetzt und 6 Stunden bei 50 °C gerührt. Man saugt den Niederschlag ab und erhält 4,5-Dihydro-5-methyl-6-(3-methylsulfonyl-4-methoxy-styryl)-pyridazin-3-on.

Analog erhält man durch Umsetzung der entsprechenden Thioether mit Wasserstoffperoxid

6-(2-Methylsulfonyl-styryl)-pyridazin-3-on
6-(3-Methylsulfonyl-styryl)-pyridazin-3-on
6-(4-Methylsulfonyl-styryl)-pyridazin-3-on
6-(2-Allylsulfonyl-styryl)-pyridazin-3-on
6-(3-Allylsulfonyl-styryl)-pyridazin-3-on
6-(4-Allylsulfonyl-styryl)-pyridazin-3-on
6-(2-Propargylsulfonyl-styryl)-pyridazin-3-on
6-(3-Propargylsulfonyl-styryl)-pyridazin-3-on
6-(4-Propargylsulfonyl-styryl)-pyridazin-3-on
6-(3-Allylsulfonyl-4-methoxy-styryl)-pyridazin-3-on
6-(4-Allylsulfonyl-3-methoxy-styryl)-pyridazin-3-on
6-(3-Propargylsulfonyl-4-methoxy-styryl)-pyridazin-3-on
6-(4-Propargylsulfonyl-3-methoxy-styryl)-pyridazin-3-on

6-(2-Methoxy-3-methylsulfonyl-styryl)-pyridazin-3-on
6-(2-Methoxy-4-methylsulfonyl-styryl)-pyridazin-3-on
6-(2-Methoxy-5-methylsulfonyl-styryl)-pyridazin-3-on
6-(2-Methoxy-6-methylsulfonyl-styryl)-pyridazin-3-on
6-(3-Methoxy-2-methylsulfonyl-styryl)-pyridazin-3-on
6-(3-Methoxy-4-methylsulfonyl-styryl)-pyridazin-3-on
6-(3-Methoxy-5-methylsulfonyl-styryl)-pyridazin-3-on
6-(3-Methoxy-6-methylsulfonyl-styryl)-pyridazin-3-on
6-(4-Methoxy-2-methylsulfonyl-styryl)-pyridazin-3-on
6-(4-Methoxy-3-methylsulfonyl-styryl)-pyridazin-3-on
6-(3,5-Dimethoxy-4-methylsulfonyl-styryl)-pyridazin-3-on
6-(3,4-Dimethoxy-5-methylsulfonyl-styryl)-pyridazin-3-on
6-(2,5-Dimethoxy-4-methylsulfonyl-styryl)-pyridazin-3-on
6-(2,3,5-Trimethoxy-4-methylsulfonyl-styryl)-pyridazin-3-on

4,5-Dihydro-6-(2-methylsulfonyl-styryl)-pyridazin-3-on
4,5-Dihydro-6-(3-methylsulfonyl-styryl)-pyridazin-3-on
4,5-Dihydro-6-(4-methylsulfonyl-styryl)-pyridazin-3-on

4,5-Dihydro-6-(2-allylsulfonyl-styryl)-pyridazin-3-on
4,5-Dihydro-6-(3-allylsulfonyl-styryl)-pyridazin-3-on
4,5-Dihydro-6-(4-allylsulfonyl-styryl)-pyridazin-3-on
4,5-Dihydro-6-(2-propargylsulfonyl-styryl)-pyridazin-3-on
4,5-Dihydro-6-(3-propargylsulfonyl-styryl)-pyridazin-3-on
4,5-Dihydro-6-(4-propargylsulfonyl-styryl)-pyridazin-3-on
4,5-Dihydro-6-(3-allylsulfonyl-4-methoxy-styryl)-pyridazin-3-on
4,5-Dihydro-6-(4-allylsulfonyl-3-methoxy-styryl)-pyridazin-3-on
4,5-Dihydro-6-(3-propargylsulfonyl-4-methoxy-styryl)-pyridazin-3-on
4,5-Dihydro-6-(4-propargylsulfonyl-3-methoxy-styryl)-pyridazin-3-on
4,5-Dihydro-6-(2-methoxy-3-methylsulfonyl-styryl)-pyridazin-3-on
4,5-Dihydro-6-(2-methoxy-4-methylsulfonyl-styryl)-pyridazin-3-on
4,5-Dihydro-6-(2-methoxy-5-methylsulfonyl-styryl)-pyridazin-3-on
4,5-Dihydro-6-(2-methoxy-6-methylsulfonyl-styryl)-pyridazin-3-on
4,5-Dihydro-6-(3-methoxy-2-methylsulfonyl-styryl)-pyridazin-3-on
4,5-Dihydro-6-(3-methoxy-4-methylsulfonyl-styryl)-pyridazin-3-on
4,5-Dihydro-6-(3-methoxy-5-methylsulfonyl-styryl)-pyridazin-3-on
4,5-Dihydro-6-(3-methoxy-6-methylsulfonyl-styryl)-pyridazin-3-on
4,5-Dihydro-6-(4-methoxy-2-methylsulfonyl-styryl)-pyridazin-3-on
4,5-Dihydro-6-(4-methoxy-3-methylsulfonyl-styryl)-pyridazin-3-on
4,5-Dihydro-6-(3,5-dimethoxy-4-methylsulfonyl-styryl)-pyridazin-3-on

4,5-Dihydro-6-(3,4-dimethoxy-5-methylsulfonyl-styryl)-pyridazin-3-on
4,5-Dihydro-6-(2,5-dimethoxy-4-methylsulfonyl-styryl)-pyridazin-3-on
4,5-Dihydro-6-(2,3,5-trimethoxy-4-methylsulfonyl--styryl)-pyridazin-3-on

4,5-Dihydro-5-methyl-6-(2-methylsulfonyl-styryl)-pyridazin-3-on
4,5-Dihydro-5-methyl-6-(3-methylsulfonyl-styryl)-pyridazin-3-on
4,5-Dihydro-5-methyl-6-(4-methylsulfonyl-styryl)-pyridazin-3-on
4,5-Dihydro-5-methyl-6-(2-allylsulfonyl-styryl)-pyridazin-3-on
4,5-Dihydro-5-methyl-6-(3-allylsulfonyl-styryl)-pyridazin-3-on
4,5-Dihydro-5-methyl-6-(4-allylsulfonyl-styryl)-pyridazin-3-on
4,5-Dihydro-5-methyl-6-(2-propargylsulfonyl-styryl)-pyridazin-3-on
4,5-Dihydro-5-methyl-6-(3-propargylsulfonyl-styryl)-pyridazin-3-on
4,5-Dihydro-5-methyl-6-(4-propargylsulfonyl-styryl)-pyridazin-3-on
4,5-Dihydro-5-methyl-6-(3-allylsulfonyl-4-methoxy-styryl)-pyridazin-3-on
4,5-Dihydro-5-methyl-6-(4-allylsulfonyl-3-methoxy-styryl)-pyridazin-3-on
4,5-Dihydro-5-methyl-6-(3-propargylsulfonyl-4-methoxy-styryl)-pyridazin-3-on
4,5-Dihydro-5-methyl-6-(4-propargylsulfonyl-3-methoxy-styryl)-pyridazin-3-on
4,5-Dihydro-5-methyl-6-(2-methoxy-3-methylsulfonyl-styryl)-pyridazin-3-on

4,5-Dihydro-5-methyl-6-(2-methoxy-4-methylsulfonyl-styryl)-pyridazin-3-on

4,5-Dihydro-5-methyl-6-(2-methoxy-5-methylsulfonyl-styryl)-pyridazin-3-on

4,5-Dihydro-5-methyl-6-(2-methoxy-6-methylsulfonyl-styryl)-pyridazin-3-on

4,5-Dihydro-5-methyl-6-(3-methoxy-2-methylsulfonyl-styryl)-pyridazin-3-on

4,5-Dihydro-5-methyl-6-(3-methoxy-4-methylsulfonyl-styryl)-pyridazin-3-on, F: 184 °C

4,5-Dihydro-5-methyl-6-(3-methoxy-5-methylsulfonyl-styryl)-pyridazin-3-on

4,5-Dihydro-5-methyl-6-(3-methoxy-6-methylsulfonyl-styryl)-pyridazin-3-on

4,5-Dihydro-5-methyl-6-(4-methoxy-2-methylsulfonyl-styryl)-pyridazin-3-on

4,5-Dihydro-5-methyl-6-(4-methoxy-3-methylsulfonyl-styryl)-pyridazin-3-on

4,5-Dihydro-5-methyl-6-(3,5-dimethoxy-4-methylsulfonyl-styryl)-pyridazin-3-on

4,5-Dihydro-5-methyl-6-(3,4-dimethoxy-5-methylsulfonyl-styryl)-pyridazin-3-on

4,5-Dihydro-5-methyl-6-(2,5-dimethoxy-4-methyl sulfonyl-styryl)-pyridazin-3-on

4,5-Dihydro-5-methyl-6-(2,3,5-trimethoxy-4-methylsulfonyl-styryl)-pyridazin-3-on

4,5-Dihydro-5-methyl-6-(2-chlor-4-methylsulfonyl-styryl)-pyridazin-3-on, F: 210-212 °C

4,5-Dihydro-5-methyl-6-[2-(2-methylsulfonylphenyl)-prop-1-enyl]-pyridazin-3-on

4,5-Dihydro-5-methyl-6-[2-(3-methylsulfonylphenyl)-prop-1-enyl]-pyridazin-3-on

4,5-Dihydro-5-methyl-6-[2-(4-methylsulfonylphenyl)-prop-1-enyl]-pyridazin-3-on

4,5-Dihydro-5-methyl-6-[2-(2-allylsulfonylphenyl)-prop-1-enyl]-pyridazin-3-on

4,5-Dihydro-5-methyl-6-[2-(3-allylsulfonylphenyl)-prop-1-enyl]-pyridazin-3-on
4,5-Dihydro-5-methyl-6-[2-(4-allylsulfonylphenyl)-prop-1-enyl]-pyridazin-3-on
4,5-Dihydro-5-methyl-6-[2-(2-propargylsulfonylphenyl)-prop-1-enyl]-pyridazin-3-on
4,5-Dihydro-5-methyl-6-[2-(3-propargylsulfonylphenyl)-prop-1-enyl]-pyridazin-3-on
4,5-Dihydro-5-methyl-6-[2-(4-propargylsulfonylphenyl)-prop-1-enyl]-pyridazin-3-on
4,5-Dihydro-5-methyl-6-[2-(3-allylsulfonyl-4-methoxy-phenyl)-prop-1-enyl]-pyridazin-3-on
4,5-Dihydro-5-methyl-6-[2-(4-allylsulfonyl-3-methoxy-phenyl)-prop-1-enyl]-pyridazin-3-on
4,5-Dihydro-5-methyl-6-[2-(3-propargylsulfonyl-4-methoxyphenyl)-prop-1-enyl]-pyridazin-3-on
4,5-Dihydro-5-methyl-6-[2-(4-propargylsulfonyl-3-methoxyphenyl)-prop-1-enyl]-pyridazin-3-on
4,5-Dihydro-5-methyl-6-[2-(2-methoxy-3-methylsulfonyl-phenyl)-prop-1-enyl]-pyridazin-3-on
4,5-Dihydro-5-methyl-6-[2-(2-methoxy-4-methylsulfonyl-phenyl)-prop-1-enyl]-pyridazin-3-on
4,5-Dihydro-5-methyl-6-[2-(2-methoxy-5-methylsulfonyl-phenyl)-prop-1-enyl]-pyridazin-3-on
4,5-Dihydro-5-methyl-6-[2-(2-methoxy-6-methylsulfonyl-phenyl)-prop-1-enyl]-pyridazin-3-on
4,5-Dihydro-5-methyl-6-[2-(3-methoxy-2-methylsulfonyl-phenyl)-prop-1-enyl]-pyridazin-3-on
4,5-Dihydro-5-methyl-6-[2-(3-methoxy-4-methylsulfonyl-phenyl)-prop-1-enyl]-pyridazin-3-on
4,5-Dihydro-5-methyl-6-[2-(3-methoxy-5-methylsulfonyl-phenyl)-prop-1-enyl]-pyridazin-3-on
4,5-Dihydro-5-methyl-6-[2-(3-methoxy-6-methylsulfonyl-phenyl)-prop-1-enyl]-pyridazin-3-on
4,5-Dihydro-5-methyl-6-[2-(4-methoxy-2-methylsulfonyl-phenyl)-prop-1-enyl]-pyridazin-3-on

4,5-Dihydro-5-methyl-6-[2-(4-methoxy-3-methylsulfonyl-phenyl)-prop-1-enyl]-pyridazin-3-on

4,5-Dihydro-5-methyl-6-[2-(3,5-dimethoxy-4-methyl-sulfonylphenyl)-prop-1-enyl]-pyridazin-3-on

4,5-Dihydro-5-methyl-6-[2-(3,4-dimethoxy-5-methyl-sulfonylphenyl)-prop-1-enyl]-pyridazin-3-on

4,5-Dihydro-5-methyl-6-[2-(2,5-dimethoxy-4-methyl-sulfonylphenyl)-prop-1-enyl]-pyridazin-3-on

4,5-Dihydro-5-methyl-6-[2-(2,3,5-trimethoxy-4-methyl-sulfonylphenyl)-prop-1-enyl]-pyridazin-3-on

4,5-Dihydro-5-methyl-6-[1-methyl-2-(2-methylsulfonyl-phenyl)-ethenyl]-pyridazin-3-on

4,5-Dihydro-5-methyl-6-[1-methyl-2-(3-methylsulfonyl-phenyl)-ethenyl]-pyridazin-3-on

4,5-Dihydro-5-methyl-6-[1-methyl-2-(4-methylsulfonyl-phenyl)-ethenyl]-pyridazin-3-on

4,5-Dihydro-5-methyl-6-[1-methyl-2-(2-allylsulfonyl-phenyl)-ethenyl]-pyridazin-3-on

4,5-Dihydro-5-methyl-6-[1-methyl-2-(3-allylsulfonyl-phenyl)-ethenyl]-pyridazin-3-on

4,5-Dihydro-5-methyl-6-[1-methyl-2-(4-allylsulfonyl-phenyl)-ethenyl]-pyridazin-3-on

4,5-Dihydro-5-methyl-6-[1-methyl-2-(2-propargyl-sulfonylphenyl)-ethenyl]-pyridazin-3-on

4,5-Dihydro-5-methyl-6-[1-methyl-2-(3-propargyl-sulfonylphenyl)-ethenyl]-pyridazin-3-on

4,5-Dihydro-5-methyl-6-[1-methyl-2-(4-propargyl-sulfonylphenyl)-ethenyl]-pyridazin-3-on

4,5-Dihydro-5-methyl-6-[1-methyl-2-(3-allylsulfonyl-4-methoxyphenyl)-ethenyl]-pyridazin-3-on

4,5-Dihydro-5-methyl-6-[1-methyl-2-(4-allylsulfonyl-3-methoxyphenyl)-ethenyl]-pyridazin-3-on

4,5-Dihydro-5-methyl-6-[1-methyl-2-(3-propargyl-sulfonyl-4-methoxyphenyl)-ethenyl]-pyridazin-3-on

4,5-Dihydro-5-methyl-6-[1-methyl-2-(4-propargyl-sulfonyl-3-methoxyphenyl)-ethenyl]-pyridazin-3-on
4,5-Dihydro-5-methyl-6-[1-methyl-2-(2-methoxy-3-methyl-sulfonylphenyl)-ethenyl]-pyridazin-3-on
4,5-Dihydro-5-methyl-6-[1-methyl-2-(2-methoxy-4-methyl-sulfonylphenyl)-ethenyl]-pyridazin-3-on
4,5-Dihydro-5-methyl-6-[1-methyl-2-(2-methoxy-5-methyl-sulfonylphenyl)-ethenyl]-pyridazin-3-on
4,5-Dihydro-5-methyl-6-[1-methyl-2-(2-methoxy-6-methyl-sulfonylphenyl)-ethenyl]-pyridazin-3-on
4,5-Dihydro-5-methyl-6-[1-methyl-2-(3-methoxy-2-methyl-sulfonylphenyl)-ethenyl]-pyridazin-3-on
4,5-Dihydro-5-methyl-6-[1-methyl-2-(3-methoxy-4-methyl-sulfonylphenyl)-ethenyl]-pyridazin-3-on
4,5-Dihydro-5-methyl-6-[1-methyl-2-(3-methoxy-5-methyl-sulfonylphenyl)-ethenyl]-pyridazin-3-on
4,5-Dihydro-5-methyl-6-[1-methyl-2-(3-methoxy-6-methyl-sulfonylphenyl)-ethenyl]-pyridazin-3-on
4,5-Dihydro-5-methyl-6-[1-methyl-2-(4-methoxy-2-methyl-sulfonylphenyl)-ethenyl]-pyridazin-3-on
4,5-Dihydro-5-methyl-6-[1-methyl-2-(4-methoxy-3-methyl-sulfonylphenyl)-ethenyl]-pyridazin-3-on
4,5-Dihydro-5-methyl-6-[1-methyl-2-(3,5-dimethoxy-4-methylsulfonylphenyl)-ethenyl]-pyridazin-3-on
4,5-Dihydro-5-methyl-6-[1-methyl-2-(3,4-dimethoxy-5-methylsulfonylphenyl)-ethenyl]-pyridazin-3-on
4,5-Dihydro-5-methyl-6-[1-methyl-2-(2,5-dimethoxy-4-methylsulfonylphenyl)-ethenyl]-pyridazin-3-on
4,5-Dihydro-5-methyl-6-[1-methyl-2-(2,3,5-tri-methoxy-4-methylsulfonylphenyl)-ethenyl]-pyridazin-3-on.

Beispiel 6

Ein Gemisch aus 26 g 4,5-Dihydro-6-(3,4-dimethoxy-styryl)-pyridazin-3-on, 25 g 2,3-Dichlor-5,6-dicyan-p-benzochinon (DDQ) und 250 ml Tetrahydrofuran wird 10 Stunden unter Stickstoff gekocht. Anschließend befreit man vom Lösungsmittel und chromatographiert den Rückstand an basischem Aluminiumoxid mit Dichlormethan als Elutionsmittel. Nach Kristallisation aus Ethanol wird 6-(3,4-Dimethoxy-styryl)-pyridazin-3-on erhalten; F: 201 °C.

Analog erhält man durch Umsetzung der entsprechenden 4,5-Dihydropyridazinone mit DDQ:

6-(2-Methylthio-styryl)-pyridazin-3-on
6-(3-Methylthio-styryl)-pyridazin-3-on
6-(4-Methylthio-styryl)-pyridazin-3-on
6-(2-Methoxy-styryl)-pyridazin-3-on
6-(3-Methoxy-styryl)-pyridazin-3-on
6-(4-Methoxy-styryl)-pyridazin-3-on
6-(2,4-Dimethoxy-styryl)-pyridazin-3-on
6-(3,4,5-Trimethoxy-styryl)-pyridazin-3-on
6-(2-Allyloxy-styryl)-pyridazin-3-on
6-(3-Allyloxy-styryl)-pyridazin-3-on
6-(4-Allyloxy-styryl)-pyridazin-3-on
6-(2-Propargyloxy-styryl)-pyridazin-3-on
6-(3-Propargyloxy-styryl)-pyridazin-3-on
6-(4-Propargyloxy-styryl)-pyridazin-3-on
6-(2-Allylthio-styryl)-pyridazin-3-on
6-(3-Allylthio-styryl)-pyridazin-3-on
6-(4-Allylthio-styryl)-pyridazin-3-on
6-(2-Propargylthio-styryl)-pyridazin-3-on
6-(3-Propargylthio-styryl)-pyridazin-3-on
6-(4-Propargylthio-styryl)-pyridazin-3-on
6-(3-Hydroxy-4-methoxy-styryl)-pyridazin-3-on
6-(4-Hydroxy-3-methoxy-styryl)-pyridazin-3-on

6-(2-Methoxy-3-methylthio-styryl)-pyridazin-3-on
6-(2-Methoxy-4-methylthio-styryl)-pyridazin-3-on
6-(2-Methoxy-5-methylthio-styryl)-pyridazin-3-on
6-(2-Methoxy-6-methylthio-styryl)-pyridazin-3-on
6-(3-Methoxy-2-methylthio-styryl)-pyridazin-3-on
6-(3-Methoxy-4-methylthio-styryl)-pyridazin-3-on
6-(3-Methoxy-5-methylthio-styryl)-pyridazin-3-on
6-(3-Methoxy-6-methylthio-styryl)-pyridazin-3-on
6-(4-Methoxy-2-methylthio-styryl)-pyridazin-3-on
6-(4-Methoxy-3-methylthio-styryl)-pyridazin-3-on

6-(3,5-Dimethoxy-4-methylthio-styryl)-pyridazin-3-on
6-(3,4-Dimethoxy-5-methylthio-styryl)-pyridazin-3-on
6-(2,5-Dimethoxy-4-methylthio-styryl)-pyridazin-3-on
6-(2,3,5-Trimethoxy-4-methylthio-styryl)-pyridazin-3-on.

Beispiel 7

Ein Gemisch von 27,4 g 4,5-Dihydro-5-methyl-6-(3,4-dimethoxy-styryl)-pyridazon-3-on, 25 g Selendioxid und 300 ml Ethanol wird 12 Stunden in einer Stickstoffatmosphäre gekocht. Danach entfernt man das Lösungsmittel unter vermindertem Druck und chromatographiert den Rückstand mit Dichlormethan an basischem Aluminiumoxid. Kristallisation des Rückstandes aus Ethanol ergibt 5-Methyl-6-(3,4-dimethoxy-styryl)-pyridazin-3-on; F: 210 - 211° C.

Analog werden durch Dehydrierung der entsprechenden 4,5-Dihydro-5-methylpyridazinone erhalten:

5-Methyl-6-(2-methylthio-styryl)-pyridazin-3-on
5-Methyl-6-(3-methylthio-styryl)-pyridazin-3-on
5-Methyl-6-(4-methylthio-styryl)-pyridazin-3-on
5-Methyl-6-(2-methoxy-styryl)-pyridazin-3-on
5-Methyl-6-(3-methoxy-styryl)-pyridazin-3-on
5-Methyl-6-(4-methoxy-styryl)-pyridazin-3-on; F: 237° C
5-Methyl-6-(2,4-dimethoxy-styryl)-pyridazin-3-on
5-Methyl-6-(3,4,5-trimethoxy-styryl)-pyridazin-3-on; F: 221° C
5-Methyl-6-(2-allyloxy-styryl)-pyridazin-3-on
5-Methyl-6-(3-allyloxy-styryl)-pyridazin-3-on
5-Methyl-6-(4-allyloxy-styryl)-pyridazin-3-on
5-Methyl-6-(2-propargyloxy-styryl)-pyridazin-3-on
5-Methyl-6-(3-propargyloxy-styryl)-pyridazin-3-on
5-Methyl-6-(4-propargyloxy-styryl)-pyridazin-3-on
5-Methyl-6-(2-allylthio-styryl)-pyridazin-3-on
5-Methyl-6-(3-allylthio-styryl)-pyridazin-3-on
5-Methyl-6-(4-allylthio-styryl)-pyridazin-3-on
5-Methyl-6-(2-propargylthio-styryl)-pyridazin-3-on
5-Methyl-6-(3-propargylthio-styryl)-pyridazin-3-on
5-Methyl-6-(4-propargylthio-styryl)-pyridazin-3-on
5-Methyl-6-(3-hydroxy-4-methoxy-styryl)-pyridazin-3-on
5-Methyl-6-(4-hydroxy-3-methoxy-styryl)-pyridazin-3-on

5-Methyl-6-(2-methoxy-3-methylthio-styryl)-pyridazin-3-on
5-Methyl-6-(2-methoxy-4-methylthio-styryl)-pyridazin-3-on
5-Methyl-6-(2-methoxy-5-methylthio-styryl)-pyridazin-3-on
5-Methyl-6-(2-methoxy-6-methylthio-styryl)-pyridazin-3-on
5-Methyl-6-[2-(3-methoxy-2-methylthio-styryl)-pyridazin-3-on
5-Methyl-6-[2-(3-methoxy-4-methylthio-styryl)-pyridazin-3-on
5-Methyl-6-[2-(3-methoxy-5-methylthio-styryl)-pyridazin-3-on
5-Methyl-6-[2-(3-methoxy-6-methylthio-styryl)-pyridazin-3-on
5-Methyl-6-[2-(4-methoxy-2-methylthio-styryl)-pyridazin-3-on
5-Methyl-6-[2-(4-methoxy-3-methylthio-styryl)-pyridazin-3-on

5-Methyl-6-(3,5-dimethoxy-4-methylthio-styryl)-pyridazin-3-on
5-Methyl-6-(3,4-dimethoxy-5-methylthio-styryl)-pyridazin-3-on
5-Methyl-6-(2,5-dimethoxy-4-methylthio-styryl)-pyridazin-3-on
5-Methyl-6-(2,3,5-trimethoxy-4-methylthio-styryl)-pyridazin-3-on.

Die nachstehenden Beispiele betreffen pharmazeutische Zubereitungen, die Verbindungen der Formel I oder ihre physiologisch unbedenklichen Salze enthalten:

Beispiel A: Tabletten

Ein Gemisch von 1 kg 4,5-Dihydro-5-methyl-6-(2-chlor-4-methylsulfinyl-styryl)-pyridazin-3-on 4 kg Lactose, 1,2 kg Kartoffelstärke, 0,2 kg Talk und 0,1 kg Magnesiumstearat wird in üblicher Weise zu Tabletten gepreßt, derart, daß jede Tablette 50 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt 10 kg 4,5-Dihydro-5-methyl-6-(2-chlor-4-methylsulfinyl-styryl)-pyridazin-3-on in üblicher Weise in Hartgelatinekapseln, so daß jede Kapsel 50 mg Wirkstoff enthält.

Beispiel D: Ampullen

Eine Lösung von 1 kg 4,5-Dihydro-5-methyl-6-(2-chlor-4-methylsulfinyl-styryl)-pyridazin-3-on in 30 l zweifach destilliertem Wasser wird steril filtriert, in Ampullen abgefüllt, unter sterilen Bedingungen lyophilisiert und steril verschlossen. Jede Ampulle enthält 20 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

Merck Patent Gesellschaft
mit beschränkter Haftung

6100 D a r m s t a d t

Patentansprüche

1. 6-Arylalkenylpyridazinone der allgemeinen Formel I,

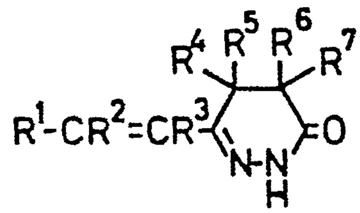

I

worin

$R^1$ eine Phenylgruppe oder eine ein- bis vierfach durch R, OH, SH, OR, SR, SO-R, $SO_2$-R, O-$SO_2$-R, S-$SO_2$-R, $NH_2$, NH-R, $NR_2$, NH-CO-R, F, Cl, Br, J, $CF_3$, $NO_2$, CN und/oder CO-$NH_2$ substituierte Phenylgruppe,

R Alkyl mit 1 bis 6 C-Atomen, Alkenyl oder Alkinyl mit 2 bis 6 C-Atomen,

$R^2$ und $R^3$ H oder Alkyl mit 1 bis 3 C-Atomen,

$R^4$ und $R^7$ jeweils H oder Alkyl mit 1 bis 4 C-Atomen und

$R^5$ und $R^6$ jeweils H oder zusammen eine C-C-Bindung bedeuten,

worin ferner

$R^1$ nur dann p-Tolyl oder p-Methoxyphenyl sein kann, wenn einer der Reste $R^2$ bis $R^7$ von H verschieden ist,

sowie ihre Salze.

2. a) 4,5-Dihydro-6-(4-methylthio-styryl)-pyridazin-3-on
   b) 4,5-Dihydro-6-(3-methoxy-4-methylthio-styryl)-pyridazin-3-on
   c) 4,5-Dihydro-6-(4-methoxy-3-methylthio-styryl)-pyridazin-3-on
   d) 4,5-Dihydro-5-methyl-6-(4-methylthio-styryl)-pyridazin-3-on
   e) 4,5-Dihydro-5-methyl-6-(4-methylsulfinyl-styryl)-pyridazin-3-on
   f) 4,5-Dihydro-6-(3-methoxy-4-methylthio-styryl)-5-methyl-pyridazin-3-on
   g) 4,5-Dihydro-6-(3-methoxy-4-methylsulfinyl-styryl)-5-methyl-pyridazin-3-on
   h) 4,5-Dihydro-6-(4-methoxy-3-methylthio-styryl)-5-methyl-pyridazin-3-on
   i) 4,5-Dihydro-6-(4-methoxy-3-methylsulfinyl-styryl)-5-methyl-pyridazin-3-on.
   j) 4,5-Dihydro-6-(2-chlor-4-methylthio-styryl)-5-methyl-pyridazin-3-on
   k) 4,5-Dihydro-5-methyl-6-(2-chlor-4-methylsulfinyl-styryl)-pyridazin-3-on
   l) 4,5-Dihydro-5-methyl-6-(2-chlor-4-methylsulfonyl-styryl)-pyridazin-3-on.

3. Verfahren zur Herstellung von 6-Arylalkenylpyridazinonen der Formel I gemäß Anspruch 1 sowie von ihren Salzen, dadurch gekennzeichnet, daß man eine Carbonsäure der allgemeinen Formel II,

$$R^1-CR^2=CR^3-CO-CR^4R^5-CR^6R^7-COOH \quad II$$

worin $R^1$ bis $R^7$ die bei Formel I angegebene Bedeutung haben, oder ein reaktionsfähiges Derivat einer solchen Carbonsäure mit Hydrazin oder einem reaktionsfähigen Hydrazinderivat umsetzt,

oder daß man ein 1,2-Diketon der allgemeinen Formel III,

$$R^1-CR^2=CR^3-CO-CO-R^4 \quad III$$

worin $R^1$ bis $R^4$ die bei Formel I angegebene Bedeutung haben, mit Hydrazin und Essigsäure oder Acethydrazid umsetzt, oder daß man ein Hydrazon der allgemeinen Formel IV,

$$R^1-CR^2=CR^3-C(=N-NH_2)-CO-R^4 \quad IV$$

worin $R^1$ bis $R^4$ die bei Formel I angegebene Bedeutung haben, mit Essigsäure umsetzt, oder daß man eine Oxoverbindung der allgemeinen Formel V mit einem Phosphonat der allgemeinen Formel VI,

$R^1-CO-R^2$ V

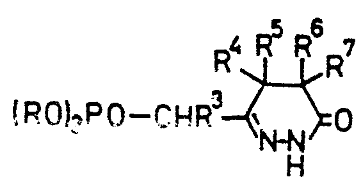

VI

worin R bis $R^7$ die bei Formel I angegebene Bedeutung haben, umsetzt, und/oder daß man Thioether der Formel I, worin mindestens einer der Substituenten SR bedeutet, zu den entsprechenden Sulfoxiden oder Sulfonen oxidiert, und daß man gegebenenfalls in ein erhaltenes Dihydropyridazinon der Formel I ($R^5 = R^7 = H$) durch Behandeln mit dehydrierenden Mitteln eine Doppelbindung in 4(5)-Stellung einführt und/oder daß man eine basische Verbindung der Formel I mit einer Säure in das zugehörige Säureadditionssalz überführt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls zusammen mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I und/oder von ihren physiologisch unbedenklichen Salzen zur Herstellung von Arzneimitteln.

7. Verwendung von Verbindungen der Formel I und/oder von ihren physiologisch unbedenklichen Salzen bei der Bekämpfung von Krankheiten.

0210530

Nummer der Anmeldung

Europäisches Patentamt

**EUROPÄISCHER TEILRECHERCHENBERICHT,**
der nach Regel 45 des Europäischen Patent-übereinkommens für das weitere Verfahren als europäischer Recherchenbericht gilt

EP 86109671.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 010 156 (MERCK)<br>* Ansprüche 1,3,4,5 *<br>-- | 1,3,4,5 | C 07 D 237/04<br>A 61 K 31/50 |
| A | GB - A - 2 122 195 (THEA)<br>* Ansprüche 1,6,8,11 *<br>---- | 1,3,5,6 | |

RECHERCHIERTE SACHGEBIETE (Int. Cl.4)

C 07 D 237/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6
Unvollständig recherchierte Patentansprüche: -
Nicht recherchierte Patentansprüche: 7
Grund für die Beschränkung der Recherche:

(Verfahren zur therapeutischen Behandlung des menschlichen oder tierischen Körpers; Art. 52(4) EPÜ)

| Recherchenort | Abschlußdatum der Recherche | Prüfer |
|---|---|---|
| WIEN | 03-11-1986 | LUX |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82